# EUROPÄISCHE PATENTSCHRIFT

(11) **EP 0 396 945 B1**
(45) Veröffentlichungstag und Bekanntmachung des Hinweises auf die Patenterteilung: **06.03.1996**
(21) Anmeldenummer: 90107488.0
(22) Anmeldetag: 19.04.1990
(51) Int. Cl.: A61B 17/39, A61M 25/10, A61M 29/02

(54) **Ballonsonde zur Thrombosierung von Gefässen**
Balloon probe for the thrombosis of vascular conduits
Sonde à ballon pour la thrombose de conduits vasculaires

(30) Priorität: 12.05.1989 DE 3915636
(43) Veröffentlichungstag der Anmeldung: 14.11.1990
(73) Patentinhaber: Scimed Life Systems, Inc., Maple Grove, Minnesota 55311-3648 (US)
(72) Erfinder: Qian, Qinghua, D-2300 Kiel 1 (DE)
(74) Vertreter: Schwan, Gerhard, Dipl.-Ing.

(56) Entgegenhaltungen:
- EP-A- 0 011 813
- EP-A- 0 182 689
- EP-A- 0 205 851
- EP-A- 0 315 982
- WO-A-82/00768
- US-A- 4 304 239
- US-A- 4 364 392
- US-A- 4 423 725
- US-A- 4 709 698

## Beschreibung

Die Erfindung betrifft eine Ballonsonde mit den Merkmalen des ersten Teiles des Anspruchs 1.

Bei der aus der EP-A-0 205 851 vorbekannten Ballonsonde der genannten Art ist die Wandung der Ballonsonde ganz oder teilweise elektrisch leitend ausgebildet. Die Wandung ist mit einer Mehrzahl von diskreten Öffnungen versehen, durch die das den Ballon aufblasende Fluid austreten kann.

Aus der EP-A-0 182 689 ist weiter bekannt, Strom durch einen Platindraht zu einer Ballonsonde zu führen.

Aus der US-A-4 364 392 ist es schließlich bekannt, Medikamente durch die poröse Ballonwandung freizugeben.

Die eingangs genannte Ballonsonde läßt eine Applikation eines Medikaments zu dem die Ballonsonde umgebenden Gewebe nicht zu.

Der Erfindung liegt damit die Aufgabe zugrunde, die vorbekannte Ballonsonde derart weiterzubilden, daß sie die Applikation eines Medikaments zu dem die Ballonsonde umgebenden Gewebe zuläßt.

Erfindungsgemäß wird diese Aufgabe durch die im kennzeichnenden Teil des Anspruchs 1 angegebenen Merkmal gelöst. Die Unteransprüche geben vorteilhafte Ausgestaltungen der Erfindung an.

Die Erfindung wird im folgenden anhand einer Zeichnung erläutert. Dabei zeigt:

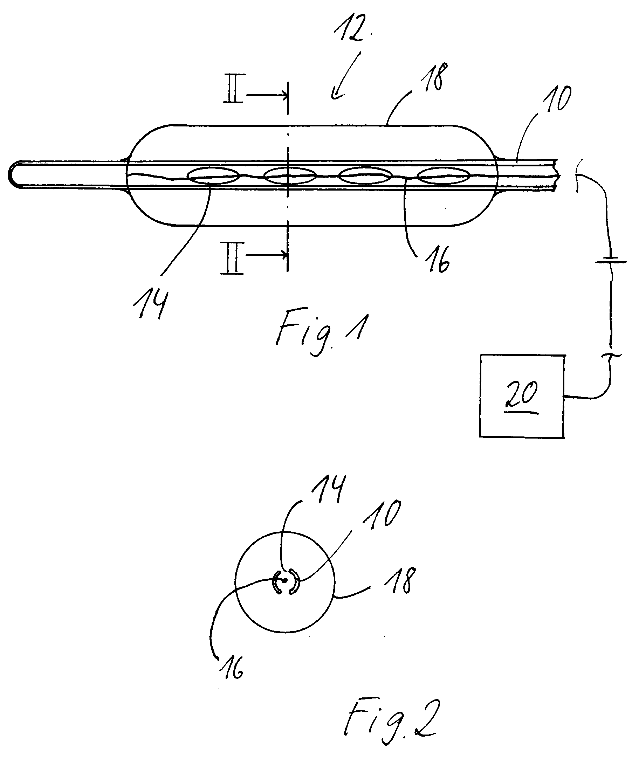

- Fig. 1: eine Schnittdarstellung durch die Ballonsonde, wobei der obere Teil des Schlauches abgeschnitten ist, und
- Fig. 2: eine Schnittdarstellung entlang der Linie II-II von Fig. 1.

Die verwendete Ballonsonde entspricht in ihrem Erscheinungsbild zunächst einer üblichen Sengstaken-Sonde: Sie besteht aus einem Schlauch 10 und einem nahe dem einen Ende des Schlauches 10 angeordneten Ballon 12, wobei der Schlauch 10 in dem Bereich, in dem er durch den Ballon 12 geführt ist, mit Öffnungen 14 versehen ist. Durch den schlauch 10 kann ein Platindraht 16 geführt werden.

Um beispielsweise Blutungen der Ösophagusvarizen zu stillen, wird die Ballonsonde in die Speiseröhre eingeführt, wobei der Ballon 12 entblockiert, also schlaff, ist. Bei Erreichen des gewünschten Ortes wird ein Fluid, beispielsweise physiologische Kochsalzlösung, in den Schlauch eingebracht, bis diese durch die Öffnungen 14 in den Ballon 12 eintritt und diesen füllt. Durch die teilpermeable Ausbildung der Ballonwandung 18 tritt eine geringe Menge des elektrisch leitenden Fluids durch die Ballonwandung 18 hindurch, so daß die Ballonwandung 18 einen definierten und genügend kleinen elektrischen Widerstand bildet.

Eine großflächige Gegenelektrode 20 wird - bei Thrombosierung von Ösophagusvarizen auf den Brustbereich - aufgebracht.

Durch Anlegen einer Spannungsquelle zwischen dem Aufweitfluid oder dem Platindraht 16 und der Gegenelektrode 20 entsteht ein Stromfluß zwischen diesen, der bei Wahl einer geeigneten Höhe der Spannung zwischen diesen die gewünschte Thrombosierung bewirkt.

Es versteht sich, daß die Vorsehung eines besonderen Drahtes 16 nicht erforderlich ist, vielmehr kann auch das in dem Schlauch befindliche elektrische leitende Fluid selbst als elektrischer Leiter verwendet werden.

Als elektrisch leitendes Fluid kann statt einer physiologischen Kochsalzlösung beispielsweise auch eine Substanz entsprechend Elektrodengel verwendet werden.

Die hier vorgeschlagene Ballonsonde kann weiterhin beispielsweise zur Behandlung postoperativer Nachblutungen bei Harnröhrenoperationen oder bei Blutungen im Enddarmbereich oder aber auch bei Blutungen aus dem Nasen-Rachenraum verwendet werden.

Es werden dem elektrisch leitenden Fluid Medikamente zugegeben, die über eine iontophoretische Gewebsdiffusion blutstillend wirken.

## Patentansprüche

1. Ballonsonde mit einem Schlauch (10) und einem daran angebrachten, mit dem Schlauchinneren in Verbindung stehenden Ballon (12) zur Thrombosierung von dicht unterhalb der Fläche einer schwer zugänglichen Körperhöhlung angeordneten Gefäßen durch Bewirken eines elektrischen Gleichstromflusses zwischen dem entblockiert in die Körperhöhlung eingebrachten und durch Einbringen eines elektrisch leitenden Aufweitfluids an Ort und Stelle geblockten Ballon und einer Gegenelektrode (20),
**dadurch gekennzeichnet, daß**
die Ballonwandung (18) für das Aufweitfluid teilpermeabel ist; und
das Aufweitfluid ein mit einem Medikament beladener, flüssiger oder gelförmiger Medikationsträger ist, der zusammen mit der Ballonwandung (18) beim Permeieren durch diese für einen definierten elektrischen Widerstand zwischen der Gegenelektrode (20) und der Fläche der Körperhöhlung sorgt, und aus dem unter dem Einfluß des thrombosierenden Gleichstromflusses Medikament iontophoretisch in das an der Ballonwandung (18) anliegende Gewebe diffundiert.

2. Ballonsonde nach Anspruch 1, dadurch gekennzeichnet, daß zur Stromzuführung zu dem Ballon (12) ein Draht (16) vorgesehen ist.

3. Ballonsonde nach Anspruch 2, dadurch gekennzeichnet, daß der Draht (16) ein Platindraht ist.

4. Ballonsonde nach einem der vorhergehenden Ansprüche, dadurch gekennzeichnet, daß das Aufweitfluid physiologische Kochsalzlösung ist.

## Claims

1. Balloon probe comprising a hose (10) and a balloon (12) attached thereto and communicating with the hose interior, for the thrombosis of vascular conduits located close under the surface of a body cavity of difficult access by passing a d.c. electric current between the balloon, which has been inserted unblocked into the body cavity and is blocked *in situ* by the introduction of an electrically conductive expansion fluid, and a counterelectrode (20),
characterised in that
the balloon wall (18) is semi-permeable to the expansion fluid; and
the expansion fluid is a liquid or gel-like medication vehicle charged with a medication, the expansion fluid together with the balloon wall (18), upon permeating said wall, ensuring a set electrical resistance between the counterelectrode (20) and the surface of the body cavity, and from which, under the effect of the thrombosing d.c. current, medicine is diffused iontophoretically into the tissue abutting the balloon wall (18).

2. Balloon probe according to claim 1, characterised in that a wire (16) is provided for conducting the current to the balloon (12).

3. Balloon probe according to claim 2, characterised in that the wire (16) is a platinum wire.

4. Balloon probe according to one of the preceding claims, characterised in that the expansion fluid is physiologically acceptable sodium chloride solution.

## Revendications

1. Sonde à ballonnet comprenant un tuyau souple (10) et un ballonnet (12) qui y est fixé et qui communique avec l'intérieur du tuyau souple, pour la thrombose de vaisseaux situés juste au-dessous de la surface d'une cavité corporelle difficile d'accès en établissant une conduction de courant électrique continu entre le ballonnet, introduit à l'état dégonflé dans la cavité corporelle et gonflé in situ par injection d'un fluide d'expansion à conduction électrique, et une contre-électrode (20), caractérisée en ce que la paroi (18) du ballonnet est partiellement perméable au fluide d'expansion, et en ce que le fluide d'expansion est un vecteur thérapeutique liquide ou en gel qui transporte un médicament et qui, conjointement avec la paroi (18) du ballonnet qu'il traverse, crée une résistance électrique définie entre la contre-électrode (20) et la surface de la cavité corporelle, et à partir duquel, sous l'effet de la conduction de courant continu de thrombose, le médicament diffuse par ionophorèse dans le tissu adjacent à la paroi (18) du ballonnet.

2. Sonde à ballonnet selon la revendication 1, caractérisée en ce qu'un fil métallique (16) est prévu pour alimenter le ballonnet (12) en courant.

3. Sonde à ballonnet selon la revendication 2, caractérisée en ce que le fil métallique (16) est un fil de platine.

4. Sonde à ballonnet selon l'une des revendications précédentes, caractérisée en ce que le fluide d'expansion est une solution physiologique de chlorure de sodium.
